(19) Europäisches Patentamt
European Patent Office
Office européen des brevets

(11) Publication number: **0 188 256 B1**

# (12) EUROPEAN PATENT SPECIFICATION

(45) Date of publication of patent specification: **21.08.91**

(51) Int. Cl.[5]: **C07C 327/00**, C07C 249/00, C07D 207/46, A61K 49/02, C07C 261/00, G01N 33/534, G01N 33/58

(21) Application number: **86100360.6**

(22) Date of filing: **13.01.86**

## (54) Metal radionuclide labeled proteins for diagnosis and therapy.

(30) Priority: **14.01.85 US 692000**

(43) Date of publication of application:
**23.07.86 Bulletin 86/30**

(45) Publication of the grant of the patent:
**21.08.91 Bulletin 91/34**

(84) Designated Contracting States:
**AT BE CH DE FR GB IT LI LU NL SE**

(56) References cited:
**EP-A- 0 135 160**

**JOURNAL OF NUCLEAR MEDICINE, vol. 25, no. 2, February 1984, pages 223-229, New York, US; R.F. SCHNEIDER et al.: N,N'-bis(S-benzoylmercaptoacetamido)ethylenediamine and propylenediamine ligands as renal function imaging agents. I. Alternate synthetic methods"**

(73) Proprietor: **NeoRx**
**410 West Harrison Street**
**Seattle Washington 98119(US)**

(72) Inventor: **Fritzberg, Alan R.**
**16703, 74th Place West**
**Edmonds, WA 98020(US)**

(74) Representative: **Glawe, Delfs, Moll & Partner**
**Patentanwälte**
**Postfach 26 01 62 Liebherrstrasse 20**
**W-8000 München 26(DE)**

**EP 0 188 256 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

### Field of the Invention

Radiolabeled compounds are important tools in medical diagnosis and treatment. Such compounds are employed in a variety of techniques including the diagnosis of deep venous thrombi, the study of lymph node pathology, and the detection, staging and treatment of neoplasms. A number of these compounds employ metal radionuclides such as Technetium-99m. When employing radionuclides for in vivo administration it is desirable that the radionuclide localize in a target organ or cancer site. Therefore, radionuclides are usually formulated to provide preferential binding to or absorption by the particular organ or tissue. There is considerable interest in being able to accurately direct a radionuclide to a preselected site to reduce background radiation directed to surrounding or distant tissue, reduce the dosage, minimize background for in vivo imaging, and minimize undesirable side effects. Toward this end, methods involving specific ligands or receptors to which the radionuclide may be conjugated are of interest.

### Description of the Prior Art

References of interest include Khaw et al., J. Nucl. Med. (1982) 23:1011; Rhodes, B.A., Sem. Nucl. Med. (1974) 4:281; Davidson et al., Inorg. Chem. (1981) 20:1629; and Byrne and Tolman, J. Nucl. Med. (1983) 24:P126. See particularly Fritzberg et al., J. Nucl. Med. (1982) 23:592; Fritzberg et al., ibid. (1981) 22:258; and Fritzberg et al., ibid. (1982) 23:P17 for descriptions of mercaptoacetyl derivatives of ethylene diamine carboxylic acid derivatives. See also U.S. Patent Nos. 4,434,151, 4,444,690, and 4,472,509.

### SUMMARY OF THE INVENTION

Metal radionuclide labeled proteins are provided for the diagnosis and treatment of a variety of pathologic conditions. Specifically, chelated radionuclide protein conjugates are employed for the diagnosis of conditions including lymph node pathology and deep venous thrombi and the detection and staging of neoplasms. Also, chelated radionuclides as protein conjugates are employed for radiotherapy of tumors.

### DESCRIPTION OF THE SPECIFIC EMBODIMENTS

Improved methods and compositions are provided related to metal radionuclide chelates, their active esters for conjugating to proteins, and the resulting peptide conjugates, as well as the use of the conjugates in radioimaging and radiotherapy.

The metal chelating compounds will be dithio, diamino- or diamidocarboxylic acids or amines or derivatives thereof, e.g., a N,N'-bis-mercaptoacetyl ω, (ω-x)-diamino carboxylic acid, (x is 1 or 2) esters capable of forming an amide bond in an aqueous medium with a polypeptide, and intermediates to the chelate. The chelating compounds are referred to as $N_2S_2$ ligands or chelates.

The compounds of this invention have the following formula:

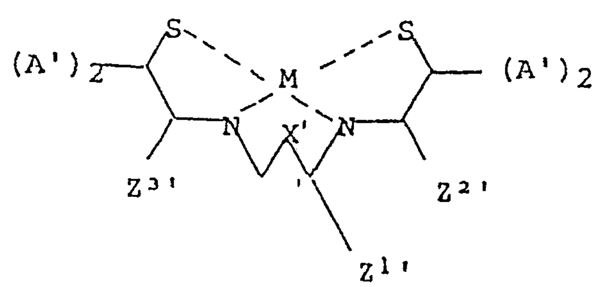

wherein:

M is a metal or metal oxide radionuclide ion capable of being chelated;

one of $Z^{1'}$, $Z^{2'}$, $Z^{3'}$, $Z^{4'}$, is $R'CW'(HNV)_n$, Y', and the others are $H_2$ or =O;

R' is an aliphatic divalent radical of from 1 to 6 carbon atoms and 0 to 2 heteroatoms;

Y' is the leaving group of an active ester, $-NH_2$, $-NHNH_2$, or a polypeptide of at least about 1000 molecular weight;

V' is of the formula R'CW';

W' is =NH or =O, with the proviso that the W' bonded to the carbon atom bonded to Y' is $H_2$ when Y' is $-NH_2$;

X' is a bond, methylene or $CHZ^{4'}$;

(A')s are the same or different and are hydrogen or lower alkyl of from 1 to 6 carbon atoms; and

n' is 0 or 1.

Furthermore, R' is an aliphatic divalent radical of from 1 to 6, usually from 1 to 3 carbon atoms, having from 0 to 1 site of aliphatic unsaturation and 0 to 2 heteroatoms, usually straight chain and preferably methylene or polymethylene of from 2 to 3 carbon atoms;

V' is R'CW, where the two R'CWs may be the same or different, usually being of from 1 to 7, more usually of from 1 to 6 carbon atoms, preferably of from 2 to 3 carbon atoms; and

Y' is an ester of an hydroxylic compound, where the ester is capable of forming an amide bond with a polypeptide in an aqueous medium without denaturation of the polypeptide; $-NH_2$; $-NHNH_2$; an amino acid, or a polypeptide usually of at least about 1000 molecular weight, more usually at least about 2000 molecular weight, generally less than about 1.6 MDal, more usually less than about 800 KDal. Of particular interest are immunoglobulins or specific binding fragments thereof.

A variety of metals may be employed as the radionuclide M.These metals include copper, e.g., $^{67}Cu$, and $^{64}Cu$, technetium, e.g. $^{99m}Tc$; rhenium, e.g. $^{186}Re$ and $^{188}Re$.

The esters are those esters which are known to provide for the reaction with a polypeptide in aqueous medium. One or another of the esters may be preferred, depending upon the particular radionuclide, the protein, and the conditions for conjugation. Common esters which find use are the o- and p-nitrophenyl, 2-chloro-4-nitrophenyl, cyanomethyl, 2-mercaptopyridyl, hydroxybenztriazole, N-hydroxysuccinimide, trichlorophenyl, tetrafluorophenyl, o-nitro-p-sulfophenyl, N-hydroxyphthalimide and the like. For the most part, the esters will be of activated phenols, particularly nitro-activated phenols and cyclic compounds based on hydroxylamine. As other hydroxylic compounds become available, these also may find use in this invention.

The polypeptide compounds may be varied widely, depending upon the nature of the use of the radionuclide. Thus, the compounds may be ligands or receptors. Ligands may include such a variety of compounds as hormones, lymphokines, growth factors, substrates, particularly compounds binding to surface membrane receptors, where the complex may remain bound to the surface or become endocytosed. Among receptors are surface membrane receptors, antibodies, enzymes, naturally occurring receptors, lectins, and the like. Of particular interest are immunoglobulins or their equivalent, which may involve Fab fragments, $F(ab')_2$, F.., T-cell receptors, etc.

The invention is furthermore directed to compounds of the following formula:

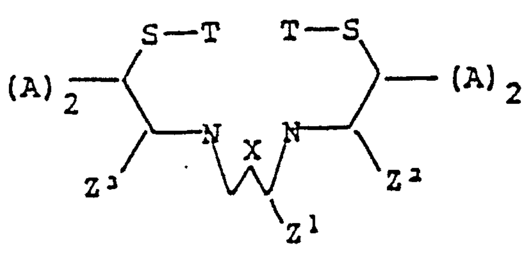

wherein:

one of $Z^1$, $Z^2$, $Z^3$ or $Z^4$ is $RCW(HNV)_nY$, and the others are $H_2$ or =O;

R is a divalent organic radical of from 1 to 6 carbon atoms and 0 to 2 heteroatoms;

W is =NH or =O, with the proviso that the W bonded to the carbon atom bonded to Y is $H_2$ when Y is $-NH_2$;

V is the same or different from RCW and comes within the definitions of RCW;

n is 0 or 1;

T is a removable protective group, hydrogen, or an alkali metal ion;

Y is an organic oxy compound forming an active ester, $-NH_2$, $-NHNH_2$, or a polypeptide of at least two amino acids;

X is a bond, methylene, or $CHZ^4$; and

the A's are the same or different and are hydrogen or lower alkyl of from 1 to 6 carbon atoms.

R is a divalent organic radical of at least one carbon atom and not more than six carbon atoms, usually from 1 to 3 carbon atoms having from 0 to 2 heteroatoms which are chalcogen (O, S) or nitrogen and is aliphatic, alicyclic, aromatic or heterocyclic, preferably aliphatic having from 0 to 2, usually 0 to 1 site of aliphatic unsaturation, e.g. ethylenic, and of from 1 to 2 carbon atoms;

T is an acyl or acylthio radical of from 2 to 10, usually 2-8 carbon atoms, either a hydrocarbyl acyl or substituted acylradical, usually aryl e.g. phenyl or alkyl, e.g. methyl, an organic sulfhydryl radical of from 1 to 10 carbon atoms, either substituted or unsubstituted hydrocarbyl, a heterocycle, particularly a chalcogen (O, S) heterocycle, an acylamidomethylene, where the acyl group is as defined above, hydrogen, sulfonato, an alkali metal ion or the two T's may be taken together to define a polyvalent metal radionuclide, as the metal ion or metal ion oxide;

substituents include, nitro, cyano, inert halo (aryl or polyhalo), carbonyl (carboxylic acid, amide and ester), and the like;

Y is hydroxyl, an oxy salt, particularly an alkali metal salt, e.g. lithium, sodium and potassium, an organic oxy compound forming an ester, usually lower alkoxy of from 1 to 6 carbon atoms or a group which permits amide formation in an aqueous medium,
particularly with a polypeptide, $-NH_2$, $-NHNH_2$, or a polypeptide of at least two amino acids and may be 2MDal (megadalton) or more; with polypeptides, particularly polypeptides over 1KDal (kilodalton) there may be more than one chelating compound bound to the polypeptide, usually not more than about one per 0.5 KDal; and

A's are the same or different and are hydrogen or lower alkyl of from 1 to 6 carbon atoms, usually of from 1 to 3 carbon atoms, particularly methyl, usually hydrogen.

The link between CW and the polypeptide will vary depending upon the nature of CW-Y. Where CW-Y is a carboxyl functionality, then the linkage will be either a carboxamide or amidine depending on whether W is =O or =NH. If, however, CW-Y defines a methyleneamine or methylenehydrazine, then reductive amination may be required with a sugar-substituted-polypeptide which has been cleaved to the oxo group, e.g., glycol cleavage with periodate. Reductive amination may be achieved by combining the oxo-substituted polypeptide with the amino- or hydrazino-substituted $N_2S_2$ ligand in the presence of a reducing agent, such as sodium cyanoborohydride.

A preferred group of compounds will have the following formula:

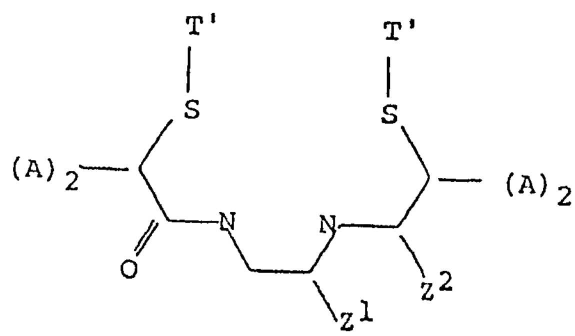

wherein all of the symbols have been defined previously except for T', and wherein:

T' is a sulfur protective group, which includes acyl, acylthio, hydrocarbylthio or substituted-hydrocarbylthio or heterocyclicthio, where the acyl and hydrocarbyl groups may be aliphatic, alicyclic, aromatic or combinations thereof and the acyl group further includes heterocyclic, wherein acyl is normally carboxyacyl; T' will generally be of from 1 to 10 carbon atoms, 2 to 10, usually 2 to 8 carbon atoms when acyl, where substituents will include non-oxo-carbonyl (carboxy), halo (aryl), particularly fluoro and chloro, cyano and nitro.

The ω, (ω-x)-diamino aliphatic carboxylic acids, particularly alkanoic acids, will be of from 4 to 10, usually from 4 to 7 carbon atoms and are known compounds, or can be readily prepared in conventional ways or as described herein. For example, vicinal dibromides may be combined with aqueous ammonia under mild conditions. The amino groups may then be derivatized by reacting the hydrochloride salt of the diamino ester, e.g., lower alkyl ester, with an α-haloacyl chloride, e.g., chloroacetyl chloride, in an inert hydrocarbon solvent, e.g., toluene, followed by substitution of the chloro groups with a mercapto group employing an appropriate derivative of hydrogen sulfide, e.g., sodium benzthiolate, sodium thioacetate, t-butyl mercaptan or the like. The ester may now be hydrolyzed to the acid and the metal chelate formed or the thioether reacted with an activated sulfonyl chloride followed by treatment with thioglycolate. Alternatively α-alkylthio substituted acyl compounds may be used with carbodiimide for acylation, followed by cleavage of the thioether with formation of disulfide and reduction of the disulfide to mercapto, as described above.

An alternative approach, employed for the 4,5-diaminopentanoate employs the readily available glutamate. After forming the 5- carboxy ester, the amino group is protected and the acid group (1-carboxyl) preferentially reduced to the alcohol. The alcohol is transformed into an active cleaving group, e.g. halide or pseudohalide, followed by displacement with a nitrogen anion, e.g. azide, which serves as an intermediate to the amino group. After catalytic reduction of the amino intermediate to amino and hydrolysis of the ester, the amino groups are acylated with S-protected α-mercaptoacyl groups. The protective groups may be, removed, exchanged or otherwise modified, e.g. by introduction of water solubilizing groups.

Various synthetic procedures may be employed for preparing the different $N_2S_2$ chelate rings. Carboxamides may be formed and reduced using aluminum or borohydrides to form the amine. Amines may be alkylated with aliphatic halides. Ethylene or propylene diamines or carboxyalkylalkylene diamines may be used to link thioglycolic acids. Other synthetic procedures may also be employed depending on the $N_2S_2$ ligand of interest.

The imidate may be employed by preparing the nitrile of the amino protected $^{\omega}$, ($^{\omega}$-x)-diaminoalkyl halide or pseudohalide by displacement with nitrile, mercaptoacylation of the deprotected amino groups as described previously and imidoester formation by conventional techniques, e.g. acidic (HCl) anh. alkanol.

The S-protective groups may be varied widely, being acyl groups, thio groups or other compound which provides protection of the thio group during the subsequent manipulations and can be readily removed without deleterious effect on the peptide conjugate.

Illustrative groups include benzoyl, acetyl, m- or p-phthaloyl, thioglycolic, o-carboxythiophenol, ethylthiocarbonate, β-mercaptopropionic, tetrahydropyranyl, sulfonato, etc. Alternatively cyclic di- or polysulfides may be formed. Disulfides may be prepared using sulfinyl halides, dinitrothiophenoxide substituted mercaptans, with mild oxidation in the presence of excess of the protective group, etc.

The protective groups may be removed in a variety of ways. Thioesters may be hydrolyzed using aqueous ammonia, sodium alkoxide in alkanol, or any conventional technique. Disulfides may be cleaved with dithiothreitol, glutathione, β-mercaptoethylamine or other conventional reagent. Cleavage of the disulfide may occur prior to or after conjugation to the polypeptide.

Depending upon the particular metal, various conditions and techniques will be employed for preparing the metal chelate. To prepare the technetium chelate, the chelating compound as carboxylate or activated ester is combined with a pertechnetate solution in the presence of a reducing agent, e.g., stannous or dithionite under conventional conditions, whereby the technetium chelate is formed as a stable salt. The rhenium chelate may be formed by reducing perrhenate with stannous ion in the presence of citrate and the $N_2S_2$ ligand. Yields are 50% or greater after 1hr at 50°C.

The chelated acid may be already esterified or esterified in accordance with conventional ways. If already esterified, a labile complex such as Tc-99m gluconate will be prepared which will allow exchange to the $N_2S_2$ activated ester ligand forming a complex suitable for protein conjugation. Alternatively, the carboxylic acid may be activated by employing a water soluble carbodiimide, e.g., EDCI, in an aqueous medium in the presence of at least a stoichiometric amount, preferably an excess of the hydroxylic compound. A suitably buffered aqueous medium may be employed. Excess carbodiimide can be converted to urea by adding acetate. The aqueous medium may then be used directly without further purification for conjugation to the polypeptide. Desirably, the polypeptide will be added to the ester containing aqueous medium at a convenient concentration at a mildly alkaline pH, generally in excess of about 7.5 and less than about 9 and the reaction allowed to proceed for a sufficient time for all of the active ester to either react with the polypeptide or be substantially completely hydrolyzed. Usually, the time will be less than about 6hr and more than about 30min, with temperatures ranging from about 0 to 50°C, usually not exceeding about 40°C. The particular conditions will be selected in accordance with the particular activated ester, the pH, the activity of the polypeptide, and the like.

It is also feasible but less preferable to conjugate the chelating agent ($N_2S_2$) to the polypeptide in the absence of the metal ion. The carboxylic acid group would be linked to the polypeptide to form a stable covalent link, e.g., an amide linkage, followed by the addition of the metal in a reduced, chelated, exchangeable form. As chelates, α- or β-dioxo compounds are useful. Conveniently, the metal ion could be added as a weakly chelated ion or in the presence of a weakly chelating group, such as a uronate, e.g. gluconate.

The subject chelates will be administered to the mammalian host, normally by injection, intravenously,

intra-arterially, peritoneally, intratumorally, or the like, depending upon the particular site at which the radionuclide is desired. Generally, from about 0.1 to 2ml will be injected into a host, depending upon the size of the host, with about 0.001 to 50$\mu$Ci/kg of host. For human hosts the dosage will usually be about 10-50mCi/70kg host, more usually about 25-35mCi/70kg host. For lower mammals, e.g. mice, 1$\mu$Ci for biodistribution studies, while up to or greater than 500$\mu$Ci for imaging studies. After administration of the radionuclide, depending upon its purpose, the host may be treated in various ways for detection or therapy.

The following examples are offered by way of illustration and not by way of limitation.

EXPERIMENTAL

Example 1

Synthesis of N,N'-bis(benzoylmercaptoacetyl)-3,4-diamino butyrate.

In a dry flask under nitrogen is placed 1.54g (0.010mole) of 3,4-diaminobutyric acid hydrochloride and 250ml of absolute ethanol. Dry HCl gas is then bubbled into the solution. The mixture is refluxed for one to two days until formation of the ethyl ester is complete. The product is then concentrated to a dry solid and the hydrochloride ester dissolved by rapid stirring at ice bath temperature in a mixture of 50ml toluene and 50ml of saturated sodium bicarbonate. To this solution is added 5.0g (0.044mole) of chloroacetyl chloride in 10ml of toluene by dropwise addition. After addition is complete, the mixture is allowed to come to room temperature and stirred for an additional 30min. Layers are separated and the aqueous portion is extracted twice with ethyl acetate. The organic layers are combined, washed with water and brine, and dried (magnesium sulfate). Removal of the solvent leaves the product as a white solid, which may be used without further purification.

A solution of 1.41g (about 4.45mmole) of the bis-chloroacetamide is prepared in 10ml of dry ethanol under nitrogen. To this is added a solution of sodium thiobenzoate in dry ethanol (prepared from sodium methoxide (0.204g of sodium, 8.87mmole, and ethanol) which is reacted with 1.23g (8.90mmole) of thiobenzoic acid). After a few minutes at room temperature, precipitation occurs. The reaction is heated to reflux for 30min. It is then allowed to cool, diluted with ethyl acetate, washed with water and brine and dried (magnesium sulfate). Removal of solvent leaves a cream-colored solid which may be recrystallized from toluene.

Example 2

Radiolabeling with Tc-99m.

1. The product prepared in Example 1 (0.1mg) is dissolved in 0.3ml of ethanol by heating and 30$\mu$l of 5N sodium hydroxide and 0.3ml of water added in succession. After heating for 15min at 95° C during which time the ethanol evaporated, an essentially aqueous solution of the hydrolyzed ligand is left. To the mixture is then added generator pertechnetate in saline (0.5ml or less) which includes about 30mCi or less of Tc-99m and 0.5mg of freshly dissolved sodium dithionite; or (2) after allowing the mixture to stand for a short period at room temperature, the mixture is heated to 95° C for an additional 15min and the pH adjusted to about 8.

2. The protected thiol, free carboxylic acid ligand of Example 1, 0.10mg, is added to 20mg of sodium gluconate and 0.010mg of $SnCl_2 \cdot 2H_2O$, pH adjusted to 5. The Tc-99m as pertechnetate is added to the mixture and the mixture heated at 95° C for 5 min.

The product mixture may be purified by preparative HPLC, using a 25cm octadecylsilane column (Altex Model 312 chromatograph; 4.6x250mm ODS ultrasphere, 5$\mu$) and eluting with 95% 0.01M sodium phosphate (pH 6) and 5% ethanol with a flow rate of 1.0ml/min. The preparations are analyzed for reduced hydrolyzed technetium on silica gel thin-layer strips.

Example 3

Formation of activated esters.

The conditions for formation of the activated esters are as follows: Into a reaction flask is introduced the carboxylic acid ligand or tracer level of metal complex carboxylate and an equimolar amount of the hydroxylic compound and a small excess, about 25% excess, of 1-ethyl-3-dimethylaminopropyl car-

bodiimide hydrochloride (ECDI) and 400μl of dimethylformamide (DMF). Upon completion of the reaction, sodium acetate is added to quench unreacted ECDI and the solution is ready for use for conjugation.

The protein to be conjugated is dissolved in 0.2M borate buffer, pH 8.5 to 9.0, to a protein concentration of about 2 to 5mg/ml. The mixture is allowed to stand at 4° C until all of the protein has dissolved. To the aqueous protein solution at a pH adjusted to 8.5-9 is added the ester solution and the pH readjusted if necessary. The resulting conjugate is then preparatively chromatographed on an HPLC gel filtration column with 0.05M phosphate, pH 7.4, buffer as eluant.

In the following study, various conditions were employed, employing activated esters of technetium chelate prepared as described above for reaction with immunoglobulin under varying conditions of time, temperature, concentration, and pH. The following Table 1 indicates the results.

TABLE 1: Reactions of Activated Esters of N,N'-bis(mercaptoacetyl)-3,4-diaminobutanoate (Tc-99m) with Immunoglobulin under Different Conditions

| Activated Ester | pH | Protein mg/ml | T(°C) | t(min) | % Labeled Protein | % Ester Hydrolyzed | % Ester Unreacted |
|---|---|---|---|---|---|---|---|
| p-nitrophenyl | 6.94 | 1.0 | 23 | 22 | 0.5 | 3.7 | 95.8 |
| | 6.94 | 1.0 | 23 | 77 | 1.7 | 8.2 | 90.1 |
| | 6.94 | 1.0 | 37 | 60 | 4.3 | 17.5 | 78.2 |
| | 6.94 | 1.0 | 37 | 105 | 6.4 | 24.9 | 68.7 |
| | 8.70 | 1.0 | 23 | 105 | 12.0 | 33.0 | 68.7 |
| | 8.70 | 1.0 | 34 | 120 | 15.0 | 69.0 | 8.0 |
| | 8.70 | 1.0 | 23 | 320 | 20.0 | 50.0 | 23.0 |
| | 9.69 | 1.0 | 23 | 120 | 13.0 | 76.0 | 7.0 |
| 2-chloro-4-nitrophenyl | 8.55 | 1.0 | 23 | 240 | 34.0 | 62.0 | 4.0 |
| | 8.55 | 1.0 | 0 | 240 | 37.0 | 60.0 | 3.0 |
| | 9.22 | 1.0 | 23 | 300 | 26.0 | 72.0 | 2.0 |
| | 9.22 | 1.0 | 0 | 330 | 30.0 | 66.0 | 4.0 |
| | 8.61 | 3.0 | 23 | 30 | 46.0 | 39.0 | 15.0 |
| | 8.61 | 3.0 | 0 | 60 | 38.0 | 46.0 | 16.0 |
| | 8.61 | 3.0 | 23 | 75 | 51.0 | 38.0 | 11.0 |
| | 8.61 | 3.0 | 0 | 100 | 44.0 | 42.0 | 14.0 |
| hydroxybenz-triazole | 6.80 | 1.0 | 23 | 30 | 8.4 | 45.4 | 46.0 |
| | 6.80 | 1.0 | 23 | 60 | 10.3 | 48.3 | 39.0 |
| | 6.80 | 1.0 | 23 | 120 | 10.8 | 53.3 | 35.8 |
| | 8.50 | 1.0 | 23 | 90 | 10.3 | 61.0 | 23.0 |
| | 8.70 | 1.0 | 5 | 120 | 32.0 | 30.0 | 32.0 |
| | 8.70 | 1.0 | 5 | 240 | 34.0 | 30.0 | 30.0 |
| | 9.25 | 1.0 | 5 | 45 | 12.0 | 33.0 | 53.0 |
| | 9.25 | 1.0 | 5 | 90 | 13.0 | 32.0 | 53.0 |
| | 9.40 | 1.0 | 23 | 60 | 4.3 | 66.0 | 21.0 |

Example 4

Synthesis of 4,5-diaminopentanoate.

To a solution of 50.5g of sodium bicarbonate in 200ml of water was added 85.0g of glutamic acid gamma-ethyl ester and the mixture cooled in an ice-salt bath. While maintaining the temperature between 0-5° C, 40g of carbobenzoxy chloride was added and the mixture stirred for 5hr followed by warming to room temperature and stirring for an additional 2hr. After extraction 2x100ml of ether, the mixture was acidified with 6N HCl to Congo red (pH 3). The separated oil was extracted with 3x100ml methylene dichloride, the combined organic layers washed with brine and water and then dried over anh. sodium sulfate. Evaporation and crystallization from 200ml carbon tetrachloride gave a yield of 46.3g (77%.) MP86-88° C.

To a solution of 46g of the above product in 45ml of THF at 35-40° C was rapidly added $BH_3$-THF (0.18mmol in 178ml). After 3hr, an aliquot on TLC (ethyl acetate hexane 4:1) showed substantially complete conversion to the alcohol.

Fifty ml of ethanol was added to the reaction mixture and the mixture evaporated to dryness. After repeating the procedure twice with 100ml of ethanol, the residue was suspended in water, extracted with ethyl acetate and the organic layer washed successively with 2x100ml of 2% aqueous bicarbonate and water, followed by drying over anh. sodium sulfate. The organic solvent was then evaporated, the residue dissolved in hexane and upon cooling gave 30.8g (71%) yield of a low-melting solid. MP86-88° C; TLC ($R_f$ ethyl acetate-hexane 0.19).

The alcohol (29.5g) prepared above was dissolved in 90ml of pyridine (0°-5° C) and 19.5g of tosyl chloride added at once. Precipitation of pyridiniumhydrochloride was observed after 1hr and the mixture stirred for 2hr more, followed by storage at 4° C overnight. The solution was poured with stirring into a liter of ice-water and the resulting solid isolated by filtration, washed with water and dried in a desiccator overnight to yield 35g (80%) of the tosyl ester. MP73°-76° C.

To the tosyl ester (22.45g) in 150ml of DMF was added 3.9g of sodium azide and the mixture heated at 50°-55° C for 3hr. At the end of this time, the DMF was removed in vacuo at 5-10 torr., cold water added and filtered. The resulting azide was dried in a desiccator overnight to yield 14.56 (91%) of the desired product. MP60°-63° C.

Into 227ml of 1N HCl-ethanol (abs) was dissolved 14g of the above azide and the solution carefully added to 1.4g of platinum oxide in a hydrogenation bottle. The mixture was hydrogenated at 50°-55° C for 48hr and the course of the reduction followed by TLC. At completion of the reaction, the catalyst was removed by filtration, the filtrate evaporated to dryness and the residue dissolved in 325ml of 6N HCl and the mixture refluxed for 36hr. After filtration and evaporation to dryness, the residue was dissolved in 100ml of water, the water evaporated and the process repeated twice. The residue was triturated with ethanol to yield 8.3g (91%) of the diamino acid product. MP>250° C.

Example 5

Synthesis of Antibody $N_2S_2$ Conjugate Using o-Nitrophenyl Disulfide Protected Ligand.

To 2.05g of the above diamino acid dissolved in 50ml of DMF was added triethylamine (3ml) and succinimidyl S-benzoyl thioglycolate (5.86g) and the mixture stirred for 15min. Dimethylformamide was removed under vacuum and 100mL of cold water was added. The precipitated oil solidified on standing. The solid was filtered, dried and crystallized from ethyl acetate. MP126-127° C.

To sodium ethoxide (140mg sodium) in 30ml ethanol was added 0.966 of the above product and the mixture stirred overnight at room temperature. After evaporating the solvent in vacuo, the residue was dissolved in glacial acetic acid, the solvent evaporated and the process repeated twice. The residue was redissolved in 30ml of glacial acetic acid and 0.77g of o-nitrophenylsulfenyl chloride added and the mixture stirred at room temperature for 24hr. The reaction was monitored by TLC (acetonitrile-water 95:5) and at completion of the reaction, the acetic acid was removed in vacuo and cold water added. The solid precipitate was filtered, washed with cold ethanol (10-15ml) and dried in vacuo for 12hr over $P_2O_5$. The yield was 1.03g (88%). MP>200° C. TLC:acetonitrile:water 95:5 $R_f$ 0.39.

To the bis-(di-o-nitrophenyldisulfide (0.293g) suspended in 50ml THF (anh.) was added N-hydroxysuccinimide (63mg) followed by dicyclohexylcarbodiimide (113mg) and the mixture stirred for 48hr at room temperature. The solution was concentrated to about 15-20ml and cooled, the precipitate removed by filtration and the filtrate diluted with 25-30ml of ethyl acetate, followed by washing the organic layer with

water. The organic layer was dried over magnesium sulfate, concentrated to 20ml and cooled. The resulting precipitate was filtered, the filterate concentrated to about 10ml and cooled to about 10°-15°C. After filtration, the filtrate was maintained at about 4°C for 2-3hr. Addition of anh. ether to the cold solution resulted in a yellow precipitate (about 95mg), followed by a second crop of about 90mg of an impure product.

The antibody conjugation reaction was contained in a final volume of 40ml: 1.8mg ($1.72x10^{-5}$ moles) bis-(di-o-nitrophenyldisulfide) $N_2S_2$ ligand, 178mg of mouse monoclonal antibody (IgG, $1.2x10^{-6}$ moles), 4.0ml of redistilled DMF, 0.05M sodium borate buffer pH 8.5. After stirring 90min at room temperature, 4.4ml of 5N sodium chloride and 1.9ml of 100mM dithiothreitol were added. After an additional 30min the reaction mixture was centrifuged to remove any particulates and the supernatant fractionated by gel filtration column chromatography. The column eluent was monitored at 280nm and the fractions containing the monomeric antibody conjugate were pooled and concentrated in an Amicon stirred cell (30,000 molecular weight cutoff). Final yield was 141mg (82%).

Example 6

Technetium-99m Labeling of Antibody-Ligand Conjugate with Tc-Tartrate.

Stannous tartrate kits were prepared from degassed solutions of 0.5ml disodium tartrate (150mg/ml) and 0.1ml stannous chloride, (1.0mg/ml in ethanol) in an evacuated vial under nitrogen atmosphere. To a stannous tartrate kit, sodium pertechnetate 0.5ml (~15mCi) was added and heated at 50°C for 10-15min. After cooling to room temperature, quality control for Tc-99m tartrate and insoluble Tc-99m was carried out on Gelman ITLC using methyl ethyl ketone and 0.01M sodium tartrate pH 7.0 eluents, respectively. Tc-99m tartrate formation was typically 98-99% with insoluble Tc-99m values ranging from 0.1 to 0.2%.

In an evacuated vial, 100μl saline, 200μl of sodium phosphate (0.2M, pH 8.0) and 200μl of antibody-ligand conjugate (1.9mg/ml) were added successively. Immediately after adding the conjugate, 250μl of Tc-99m tartrate (~3 to 5mCi was added and heated at 50°C for 1hr. Percent technetium bound to protein and the formation of pertechnetate were determined by ITLC using 50% MeOH:10% ammonium acetate (1:1) and 1-butanol eluents, respectively. Technetium incorporation typically ranged from 70-88° on a ligand -Ab conjugate with a ligand per antibody ratio of 1.5 to 3.0.

TABLE 2: Comparative Biodistribution of Tc-99m and Iodine-125 Anti-melanoma Antibody 9.2.27 in Mice Bearing Melanoma Tumors From FEMX Cell Line.

| Organ | Tumor | Liver | Spleen | Lung | Stomach | Thyroid | Kidney |
|---|---|---|---|---|---|---|---|
| Tc-99m | 5.78* ±0.32 | 1.54 ±0.19 | 1.34 ±0.14 | 1.79 ±0.67 | 0.26 ±0.15 | 0.61 ±0.05 | 1.72 ±0.12 |
| I-125 | 3.97 ±0.61 | 1.07 ±0.17 | 1.59 ±0.03 | 1.81 ±0.12 | 2.99 ±1.89 | 7.79 ±4.50 | 1.33 ±0.04 |

* Data are mean ±S.D. percent injected dose per gram for three mice at 48hr post injection.

The method of Hwang et al., Cancer Res. (1985) 45:4150-4155 was employed.

Example 7

Labeling of Antibody with Preformed Tc-99m Pentanoyl $N_2S_2$ Chelate.

A Tc-99m chelated derivative was conjugated to an antibody as follows. Tc-99m (75mCi) chelated by N,N'-bismercaptoacetyl 4,5-diaminopentanoic acid was prepared by dithionite reduction of Tc-99m pertechnetate at basic pH with 25$\mu$g of the $N_2S_2$ ligand. The acid was activated by adding the above complex at pH 7 in 0.5ml water to 100$\mu$l of water:acetonitrile (1:9) containing 3.0mg of 2,3,5,6-tetrafluorophenol and 100$\mu$l of $H_2O$:acetonitrile (1:9) containing 7.5mg of 1-cyclohexyl-3-(2-morpholinoethyl)carbodiimide(morpho CDI) added. After storing for 18hr at room temperature, the mixture was purified using a Baker-10 SPE reversed phase $C_{18}$ column. The column was conditioned with 2ml of ethanol followed by washing with HPLC grade water. The reaction mixture was then added to the column, the column washed 4x with 2ml volumes of 10% methanol in 0.01M sodium phosphate, pH 7.0 and the ester complex eluted with 2.5ml portions of acetonitrile. The first eluent contained 8.5mCi and the second 0.18mCi. The yield was 86% after accounting for decay.

To a 2ml vial was added 4.5 mCi of activated ester complex in acetonitrile, the solvent evaporated in a nitrogen stream, and 0.40ml of sodium borate (0.5M, pH 9.0) added. With stirring, 30$\mu$l (9.14mg/ml) of anti-melanoma antibody (9.2.27) was added. The final protein concentration was 0.52mg/ml. The reaction was followed with TLC using Gelman ITLC SG strips and eluting with 50% aqueous methanol:10% ammonium acetate (1:1), indicating that 47% protein bound Tc-99m at 15min and 59% at 30min at room temperature. The Tc-99m labeled protein was purified by Centricon-10k filter centrifugation. A sample of 92.4% protein bound Tc-99m showed 84.0% binding to FEMX melanoma cells.

Example 8

Preparation of Re-186 4,5-dimercaptoacetamidopentanoyl-antibody (anti-melanoma antibody 9.2.27).

In an evacuated vial is combined 100$\mu$l of $H_2O$, 100$\mu$l acetonitrile, 100$\mu$l of citrate solution (28.8mg, $1.5 \times 10^{-4}$ mol), 50$\mu$l of ligand (tetrafluorophenyl 4,5-di-(tetrahydropyranylmercaptoacetamido)pentanoate (0.40mg; $6.5 \times 10^{-7}$ mol), 50$\mu$l of stannous chloride (0.5mg, $2.6 \times 10^{-6}$ mol), and 50$\mu$l of Re-186 perrhenate in acetonitrile (4.25$\mu$g, $2.3 \times 10^{-8}$ mol). The mixture is heated at 50°C for 1hr and then 0.30ml of 1N NaOH is added.

The tetrafluorophenyl ester product of the Re-186 $N_2S_2$ complex is purified on a $C_{18}$ Baker-10 SPE column. After application to the column, impurities are washed off with 2x3ml of $H_2O$ and 4x3ml of 10% $CH_3OH$/.01M phosphate, pH 7. The product is eluted with 2ml of acetonitrile and then the solution is reduced to dryness under a stream of nitrogen. Yields of product are about 60%.

Conjugation of Re-186 $N_2S_2$ complex is done by addition of antibody (160$\mu$l of 5mg/ml) (Morgan et al., Hybridoma (1981) 1:27), in 340$\mu$l of borate buffer (0.5M, pH 9). After 30min at room temperature, 58% of the radioactivity was protein bound. Immunoreactivity determined by binding of radioactivity to FEMX melanoma cells was 80% after correction for nonprotein bound material.

Example 9

Synthesis of Imidate form of $N_2S_2$ Ligand,
Conjugation to Antibody and Radiolabeling with Tc-99m.

2,3-(Bis-carbobenzyloxy)diaminopropan-1-ol [2]

A 500mL hydrogenation bottle was charged with 55g (0.25 mol) of 2,3-dibromopropanol (Aldrich) and 300mL of 28-30% aqueous $NH_4OH$ solution. The mixture was stoppered with an internal thermometer and heated to 75-85°C while shaking on a Parr shaker for 23hr. When cool, shaking was stopped and the mixture was carefully opened. The mixture was evaporated to a volume of 50mL by passing $N_2$ gas through it while heating on an oil bath. While hot, 50mL of EtOH was added and the mixture was allowed to cool. The hydrogen bromide salt of 2,3-diaminopropan-1-ol was collected by filtration and dried in vacuo to yield 50g of hard chunks of white solid which was used without further purification.

A solution of 25g of the crude salt in 110mL of 4N NaOH was cooled to 0° (ice bath), and to the solution was added a solution of 31.4mL (0.22mol, 37.5g) of benzylchloroformate in 100mL of $CH_2Cl_2$. The mixture was stirred rapidly for 30min at 0° and 16hr at room temperature. The $CH_2Cl_2$ phase was collected, washed with 75mL of brine, dried ($MgSO_4$), filtered and concentrated. The resulting solid was washed with 100mL of $Et_2O$, collected by filtration, and dried under vacuum to give 10.7g (24%) of 2 as a white solid which could be recrystallized from $CHCl_3$/hexane to give tiny needles. MP119-120°C.

2,3-(Bis-carbobenzyloxy(diaminopropyl-1-methanesulfonate [3]

To a suspension of 10.68g (30mmol) of 2 and 6.27mL (4.55g, 45mmol) of $Et_3N$ in 150mL of $CH_2Cl_2$ cooled to 0° under $N_2$ atmosphere was added 2.55mL (3.78g, 33mmol) of methanesulfonyl chloride, and the mixture was stirred for 30min at 0° C. The resulting clear solution was washed successively with 75mL of 5% HCl, 75mL of $H_2O$, 75mL of 5% $NaHCO_3$, and 75mL of sat. aq. NaCl (all chilled in ice). The $CH_2Cl_2$ phase was dried ($MgSO_4$), filtered, concentrated, and crystallized from $CHCl_3$/hexane to yield 12.33g (94%) of white crystals. MP92-93°.

3,4-(Bis-carbobenzyloxy)diaminobutyronitrile [4]

A mixture of 6.56g (15mmol) of 3, 1.08g (16.5mmol) of KCN, 0.40g (1.5mmol) of 18-crown-6, and 75mL of anhydrous acetonitrile (stored over 3A molecular sieves) was refluxed in a nitrogen atmosphere for 19hr. When cool, the mixture was partitioned between 100mL of 10% $NaHCO_3$ solution and 200mL of $CH_2Cl_2$. The $CH_2Cl_2$ layer was washed successively with 100mL portions of 5% HCl, water and brine. The $CH_2Cl_2$ phase was dried ($MgSO_4$), filtered, and concentrated to give 5.47g of brown oil. Two recrystallizations from $CHCl_3$/hexane yielded 2.68g of 4 as a white solid. MP111-112° C.

3,4-Diaminobutyronitrile dihydrogeniodide salt [5]

To 3.38g (13.3mmol) of $I_2$ in a 100mL flask under $N_2$ atmosphere was added 5.42mL (3.87g, 26.5mmol) of hexamethyldisilane. The mixture was immersed in a 45-50° C oil bath until solid $I_2$ dissolved (30min). The temperature was raised to 100° and held for 5min until the color disappeared. The solution was cooled to 0° with an ice bath and diluted with 13.3mL of $CH_2Cl_2$. To the 0° C solution was added dropwise over 5min a solution of 1.96g (5.3mmol) of 4 in 13.3mL of $CH_2Cl_2$. The cooling bath was removed and the mixture was stirred in the dark for 3hr at room temperature. To the mixture was added 2.15mL (1.70g, 53mmol) of MeOH and stirring was continued overnight (16hr). The mixture was cooled to 0° C and the solid was collected by filtration and dried under vacuum to give 1.75g (100%) of a tan solid 5 which was characterized as its dibenzoyl derivative.

3,4-Dibenzoylmercaptoacetamidobutyronitrile [6]

To a mixture of 3.27g (10mmol) of 5, 7.33g (25mmol) of N-succinimidyl S-benzoylmercaptoacetate and 10mL of DMF was added at 0° C under $N_2$ atmosphere, 3.48mL (2.52g, 25mmol) of triethyl amine. The cooling bath was removed and the mixture was stirred for 1hr. The mixture was diluted with 50mL of 5% HCl solution and extracted with 2x50mL of $CH_2Cl_2$. The combined $CH_2Cl_2$ phases were washed with 100mL of 5% $NaHCO_3$ solution, dried ($MgSO_4$), filtered, and concentrated in vacuo to yield 6.75g of a purple tinted solid.

Purification was accomplished by chromatography (silica gel, EtOAc) and crystallization of purified fractions ($CHCl_3$/hexane) to give 3.20g (70%) of white solid. Mp125-127° C.

3,4-Bis-methyldithioacetamidobutyronitrile [7]

To a suspension of 455mg (1.0mmol) of 6 in 6mL of EtOH at room temperature under $N_2$ atmosphere was added 2.2mL of 1N aqueous NaOH. The mixture was stirred at room temperature for 1.6hr, and to the resulting clear solution was added 226$\mu$l of methyl methanethiolsulfonate. The mixture was stirred for 3hr and partitioned between 20mL of pH 7 buffer solution and 2x20mL of $CH_2Cl_2$. The combined aqueous layers were dried ($MgSO_4$), filtered, and concentrated to give 591mg of pink residue. Purification by silica gel chromatography (EtOAc) and crystallization from $CHCl_3$/hexane gave a total of 217mg (64%) of white amorphous solid 7. MP121-123° C.

Methyl 3,4-bis-methyldithioacetamidobutyrimidate hydrogen chloride salt [1]

A suspension of 141mg (0.41mmol) of 7 in 1.66mL of MeOH and 4.15mL of $Et_2O$ was cooled to -20° C ($CO_2$/$CCl_4$), and HCl gas was passed through the mixture via septum inlet for 5min, until most of the solids had dissolved and the solution was saturated with HCl. The mixture was placed in the freezer in a desiccator for 66hr, and then concentrated under vacuum to produce a white foamy solid. The solid was broken up, washed with three portions of anhydrous $Et_2O$, dried under vacuum to give 111mg (66%) of 1 as

a off-white solid which decomposed on heating and also decomposed after several days in a freezer.

Preparation of Antibody - Methyl 3,4-bis-methyldithioacetamidobutyrimidate Conjugate

A 2mg/ml stock solution of the $N_2S_2$ ligand was prepared in dry acetonitrile. The solution was standardized by determining the disulfide content using 2-nitro-5-thiosulfobenzoate (Thannhauser et al., Anal. Biochem. (1984) 138:181) and the ligand concentration was found to be 5.30mM.

For conjugation to mouse monoclonal antibody, 0.16ml of $N_2S_2$ ligand-acetonitrile solution was added to the reaction vial and the solvent removed with a stream of dry nitrogen. Antibody (0.62ml of 8.1mg/ml solution) and 1.0ml of 0.2M sodium bicarbonate buffer pH 9.5 were mixed and then added to the reaction vessel containing the dried ligand. After stirring 30min at room temperature, the entire solution was added to a fresh vial containing the same amount of dried ligand and the solution stirred another 30min. The conjugated antibody was purified by Sephadex G-25 chromatography in 50mM sodium phosphate pH 7.5, 0.5M sodium chloride. The protein containing fractions were pooled and concentrated in an Amicon stirred cell to a concentration of about 2mg/ml. The solution was made 50mM in glutathione, stirred 25min, then purified by Sephadex G-25 gel filtration and concentrated as before. The final solution (1.7mg/ml) was stored at 4°C until use.

Radiolabeling of Antibody - Methyl 3,4-bis-methyldithioacetamidobutyrimidate Conjugate

Tc-99m tartrate was prepared in a total volume of 1.1ml of degassed water with 100µg $SnCl_2$, 9% (v/v) ethanol, 75mg disodium tartrate, and 3.2mCi sodium (Tc-99m) pertechnetate. The solution was heated at 50°C for 15min. To a separate vial was added 100µl of the Tc-99m tartrate solution, 100µl of 0.2M sodium bicarbonate, pH 10, and 100µg of the antibody conjugate. The total volume was then adjusted to 0.5ml with 0.15M sodium chloride and the solution incubated at 50°C for 60min. Analysis by HPLC (TSK column, 0.2M sodium phosphate pH 7.4, 0.15M sodium chloride) showed 95% of the Tc-99m was associated with the antibody conjugate.

Example 10

Preparation of S-terephthaloylsubstituted $N_2S_2$ Ligand.

The mono-tert-butyl ester of terephthalic acid 1 was prepared by the method of Buckle and Smith, J. Chem. Soc. (1971) 54:2821.

Succinimidyl ester 2 was prepared by stirring 1 with 1.2 molar equivalents of N-hydroxysuccinimide and 1.3 molar equivalents of 1,3-dicyclohexylcarbodiimide in dry THF at room temperature for 14-16hr. Thin layer chromatographic analysis indicated the reaction had gone to completion. The dicyclohexylurea was then removed by filtration and the resulting liquid was concentrated in vacuo to yield 2 as a white solid. Final purification of 2 was accomplished by flash chromatography.

The thioester 3 was prepared by dissolving 1.0 molar equivalents of mercaptoacetic acid and 2.0 molar equivalents of 4-dimethylaminopyridine in dry THF. The succinimidyl ester 2 was added to the stirring solution. After stirring for 5hr the reaction was complete as indicated by thin layer chromatographic analysis. The THF was removed in vacuo and the residue was dissolved in $CH_2Cl_2$. The solution was then washed with dilute aqueous HCl and dried over anhydrous $MgSO_4$. Filtration and evaporation of the solvent gave 3 as a colorless oil which solidified upon standing.

The succinimidyl ester 4 was prepared by the method of Subramanian (R.F. Schneider et al., J. Nucl. Med., (1984) 25:223-229).

The carboxylic acid 5 was prepared by dissolving 4,5-diaminopentanoic acid dihydrochloride salt in 1:4 $H_2O$:$CH_3CN$ containing 3.0 molar equivalents of triethylamine and subsequently adding 2.0 molar equivalents of the succinimidyl ester 4. After stirring for 14-18hr at room temperature, TLC analysis showed the reaction to be complete and the solvent was removed in vacuo. The residue was dissolved in ethyl acetate and washed with dilute aqueous HCl, water, and brine. The ethyl acetate layer was then dried over anhydrous $Na_2SO_4$. After filtration and removal of the solvent, a waxy solid was obtained which was recrystallized from a mixture of ethyl acetate and hexane to give 5 as a white solid.

Tetrafluorophenyl ester 6A was prepared by dissolving 5, along with 1.2 molar equivalents of 2,3,5,6-tetrafluorophenol in dry THF. 1,3-dicyclohexylcarbodiimide (1.2 molar equivalents) was added to the mixture and the mixture was stirred for 12-15hr. Analysis by thin layer chromatography indicated the reaction was complete. The dicyclohexylurea was removed and by filtration and the solvent was removed in vacuo. The

residue was purified by flash chromatography to yield the ester 6A as a white solid.

Succinimidyl ester 6B was prepared by dissolving 5 along with 1.2 molar equivalents of N-hydroxysuccinimide in dry THF. 1,3-dicyclohexylcarbodiimide (1.2 molar equivalents) was added to the mixture and the mixture was stirred at room temperature for 14-18hr. Thin layer chromatographic analysis indicated the reaction had gone to completion. The dicyclohexylurea was removed by filtration and the solvent was removed in vacuo. The residue was dissolved in ethyl acetate and washed with water. The ethyl acetate solution was dried over anhydrous $Na_2SO_4$. The drying agent was removed by filtration and the solvent was removed in vacuo. The resulting residue was purified by flash chromatography to yield the succinimidyl ester 6B as a white solid.

Removal of the tert-butyl protecting groups was accomplished by dissolving the tetrafluorophenyl ester 6A in $CH_2Cl_2$ and treating the solution with excess trifluoroacetic acid, initially at 0° C, then stirring to room temperature for 3hr. Thin layer chromatographic analysis showed that the reaction had gone to completion. The solvent and excess trifluoroacetic acid were then removed in vacuo to yield a white to colorless solid which was recrystallized from $CH_3CN/H_2O$ to give 7A as a white powder.

In the case of the succinimidyl ester 6B, the tert-butyl protecting groups were removed as described above for compound 6A. It was necessary, however, to purify the product 7B by flash chromatography.

These reaction sequences were also carried out starting with the mono tert-butyl ester of isophthalic acid to obtain the analogous meta isomers of the products described above.

Example 11

Conjugation of N-hydroxysuccinimidyl 4,5-diterephthaloylmercaptoacetamidopentanoate to IgG antibody.

The conjugation was carried out in a total volume of 2.0ml and contained 480μg ($7.1x10^{-7}$ moles) $N_2S_2$ ligand active ester 6B, 0.2ml redistilled DMF (10%), 0.15M sodium chloride, 0.05M sodium borate, pH 8.5, and 10.0mg mouse monoclonal antibody ($6.7x10^{-8}$ moles). After stirring 90min at room temperature the reaction was fractionated by gel filtration over Sephadex G-28 in 0.05M sodium phosphate buffer pH 7.5 with 0.15M sodium chloride. The excluded volume containing the conjugated antibody was collected. To remove any residual non-protein material the conjugate was dialyzed 18hr against 0.05M sodium phosphate, pH 7.5, with 0.15M sodium chloride. Final yield of protein was 100%.

Example 12

Tc-99m Labeling of 4,5-diterephthalylmercaptoacetamidopentanoyl-IgG Antibody Conjugate.

To 120μl saline 200μl of 0.2M sodium phosphate buffer, pH 8, and 80μl of the terephthaloyl sulfur protected $N_2S_2$ conjugate (4.66mg/ml), 250μl of the Tc-99m tartrate (~4mCi) prepared as previously described was added. The reaction mixture was heated at 50° C for 1hr which resulted in a Tc-uptake of 90%.

Following the above procedure the isophthaloyl analog could also be prepared.

It is important that the resulting product provide for maximum formation of the radionuclide conjugates. In addition, there is the concern about the time, since the radioisotopes do decay with time. Thus, by using the compounds of the subject invention, one can rapidly conjugate proteins to provide radionuclide substituted reagents for use in vivo. The reagents can be provided in pure form, good yield, and the radionuclide metal is stably maintained as a chelate with the protein for use in vivo. Thus, one can safely direct the radionuclide to a desired site, where only low levels of radioactivity will be non-specifically directed and bound.

**Claims**

**Claims for the following Contracting States: BE, CH, DE, FR, GB, IT, LI, LU, NL, SE:**

**1.** A compound of the formula:

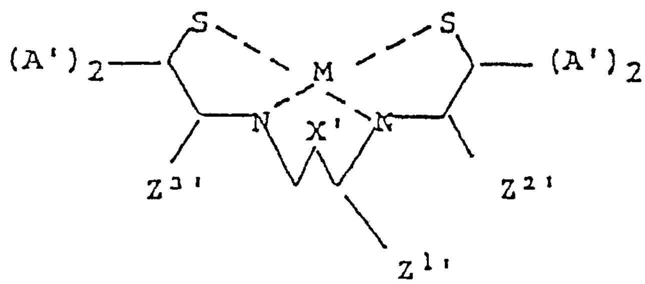

wherein:

M is a metal or metal oxide radionuclide ion capable of being chelated;

one of $Z^{1'}$, $Z^{2'}$, $Z^{3'}$, $Z^{4'}$, is $R'CW'(HNV)_n$, Y', and the others are $H_2$ or =O;

R' is an aliphatic divalent radical of from 1 to 6 carbon atoms and 0 to 2 heteroatoms;

Y' is the leaving group of an active ester, $-NH_2$, $-NHNH_2$, or a polypeptide of at least about 1000 molecular weight;

V' is of the formula R'CW';

W' is =NH or =O, with the proviso that the W' bonded to the carbon atom bonded to Y' is $H_2$ when Y' is $-NH_2$;

X' is a bond, methylene or $CHZ^{4'}$;

(A')s are the same or different and are hydrogen or lower alkyl of from 1 to 6 carbon atoms; and

n' is 0 or 1.

2. A compound according to Claim 1, wherein Y' is a polypeptide.

3. A compound according to Claim 2, wherein Y' is an immunoglobin or specific binding fragment thereof.

4. A compound of the formula:

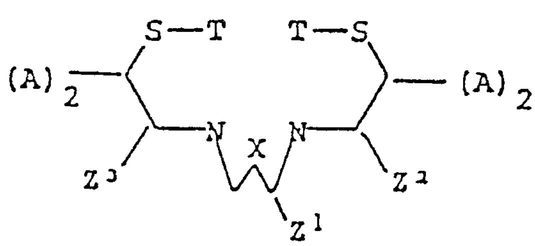

wherein:

one of $Z^1$, $Z^2$, $Z^3$, $Z^4$, is $RCW(HNv)_nY$, and the others are $H_2$ or =O;

R is an divalent radical of from 1 to 6 carbon atoms and 0 to 2 heteroatoms;

W is =NH or =O, with the proviso that the W bonded to the carbon atom bonded to Y is $H_2$ when Y is $-NH_2$;

V is the same or different from RCW and comes within the definitions of RCW;

n is 0 or 1;

T is a removable protective group, hydrogen, or an alkali metal ion;

Y is an organic oxy compound forming an active ester, $-NH_2$, $-NHNH_2$, or a polypeptide of at least two amino acids;

X is a bond, methylene or $CHZ^4$; and

the A's are the same or different and are hydrogen or lower alkyl of from 1 to 6 carbon atoms.

**5.** A compound according to Claim 4, wherein X is a bond.

**6.** A compound according to Claims 4 or 5, wherein Y is an activated phenoxy group.

**7.** A method for preparing a radiolabeled polypeptide which comprises:

combining in an aqueous medium a polypeptide and a compound of the formula according to Claim 1, wherein Y is the leaving group of an active ester capable of forming an amide or amidine link with said polypeptide, or $NH_2$ or $NHNH_2$, with the proviso that when Y is $NHNH_2$ or $-NH_2$, said polypeptide has an oxo-group as a result of glycol cleavage of a sugar bound to said polypeptide and said combining is under reductive amination conditions;

wherein said compound forms a stable covalent link to said polypeptide with the substantial absence of unchelated radionuclide.

**8.** A method for preparing a radiolabeled polypeptide which comprises:

combining in an aqueous medium a compound according to anyone of Claims 4 to 6, wherein T is an alkali metal ion or a sulfur protective group and Y is said polypeptide, and a metal radionuclide in a reduced chelated exchangeable form;

whereby said radionuclide becomes chelated by said compound; and

washing said radionuclide containing compound to remove unchelated radionuclide bound to said compound.

**9.** A compound of the formula

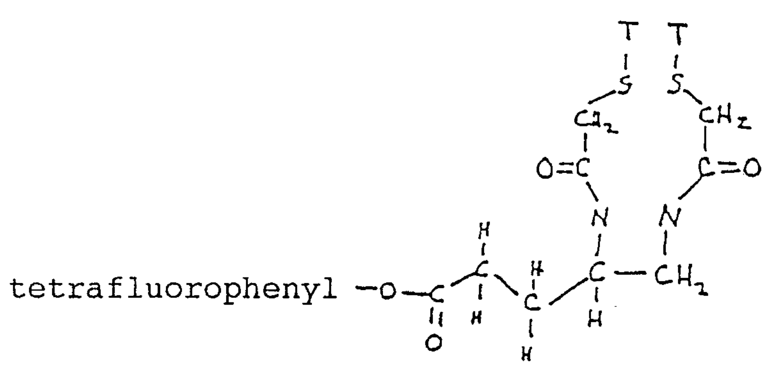

in which T is a sulfur protective group.

10. A compound of the formula:

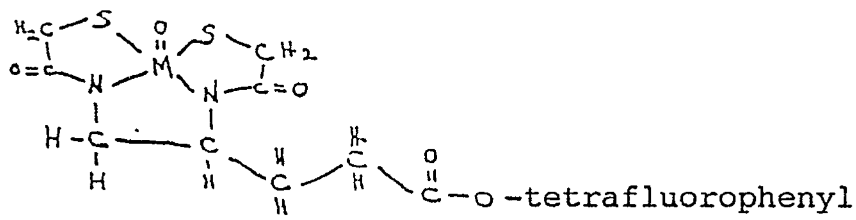

wherein M represents a metal radionuclide selected from Tc-99m and Re-186.

**Claims for the following Contracting State: AT:**

1. A method of making a compound of the formula:

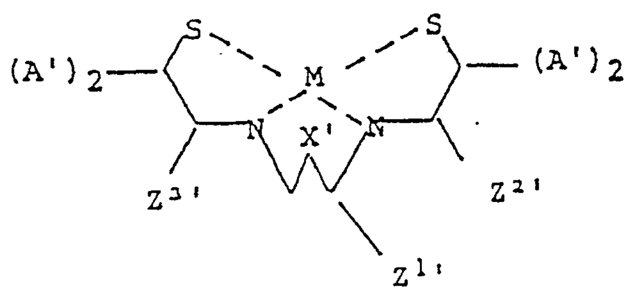

wherein:

M is a metal or metal oxide radionuclide ion capable of being chelated;

one of $Z^{1'}$, $Z^{2'}$, $Z^{3'}$, $Z^{4'}$, is $R'CW'(HNV)_{n'}$ Y', and the others are $H_2$ or $=O$;

R' is an aliphatic divalent radical of from 1 to 6 carbon atoms and 0 to 2 heteroatoms;

Y' is the leaving group of an active ester, $-NH_2$, $-NHNH_2$, or a polypeptide of at least about 1000 molecular weight;

V' is of the formula R'CW';

W' is $=NH$ or $=O$, with the proviso that the W' bonded to the carbon atom bonded to Y' is $H_2$ when Y' is $-NH_2$;

X' is a bond, methylene or $CHZ^{4'}$;

(A')s are the same or different and are hydrogen or lower alkyl of from 1 to 6 carbon atoms; and

n' is 0 or 1.

comprising the step of chelating a metal or metal oxide radionuclide ion with a precursor of said compound.

2. A method according to Claim 1, wherein Y' is a polypeptide.

3. A method according to Claim 2, characterized in that Y' is an immunoglobin or specific binding fragment thereof.

4. A method for preparing a radiolabeled polypeptide which comprises:

combining in an aqueous medium a polypeptide and a compound of the formula according to Claim 1, wherein Y is the leaving group of an active ester capable of forming an amide or amidine link with said polypeptide, or $NH_2$ or $NHNH_2$, with the proviso that when Y is $NHNH_2$ or $-NH_2$, said polypeptide has an oxo-group as a result of glycol cleavage of a sugar bound to said polypeptide and said combining is under reductive amination conditions;

wherein said compound forms a stable covalent link to said polypeptide with the substantial absence of unchelated radionuclide.

5. A method for preparing a radiolabeled polypeptide which comprises: washing a radionuclide containing compound obtained according to Claim 1 to remove unchelated radionuclide bound to said compound.

6. A method according to anyone of the preceding claims, characterized in that a compound of the formula:

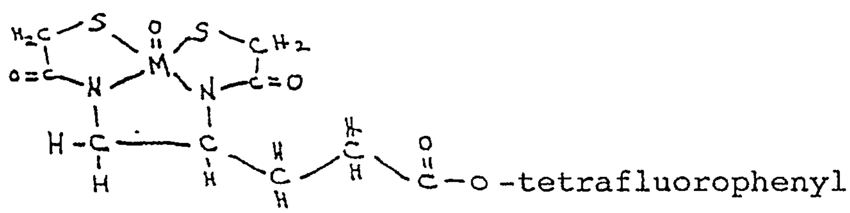

is prepared,

wherein M represents a metal radionuclide selected from Tc-99m and Re-186.

## Revendications

**REVENDICATIONS pour les Etats contractants suivants: BE, CH, DE, FR, GB, IT, LI, LU, NL, SE.**

1. Composé de formule

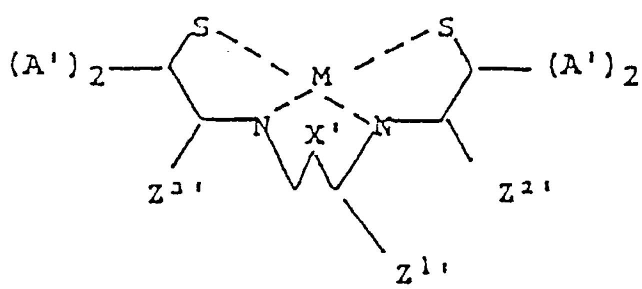

dans laquelle
M est un ion de radionucléide de métal ou d'oxyde métallique pouvant être chélaté;
l'un de $Z^{1'}$, $Z^{2'}$, $Z^{3'}$, $Z^{4'}$ est $R'CW'(HNV)_{n'}Y'$ et les autres sont $H_2$ ou =O;
R' est un radical divalent aliphatique de 1 a 6 atomes de carbone et de 0 à 2 hétéroatomes;
Y' est le groupement labile d'un ester activé, $-NH_2$, $-NHNH_2$ ou un polypeptide de poids moléculaire d'au moins environ 1000;
V' a la formule R'CW';
W' est =NH ou =O, pourvu que le groupement W' lié à l'atome de carbone lié a Y' soit $H_2$ quand Y' est $-NH_2$;
X' est une liaison, un groupement méthylène ou $CHZ^{4'}$;
les radicaux A' sont identiques ou différents et sont des atomes d'hydrogène ou des groupements alkyle inférieur ayant de 1 à 6 atomes de carbone; et

n' est 0 ou 1.

**2.** Composé selon la revendication 1, dans lequel Y' est un polypeptide.

**3.** Composé selon la revendication 2, dans lequel Y' est une immunoglobuline ou un fragment à liaison spécifique de celle-ci.

**4.** Composé de formule

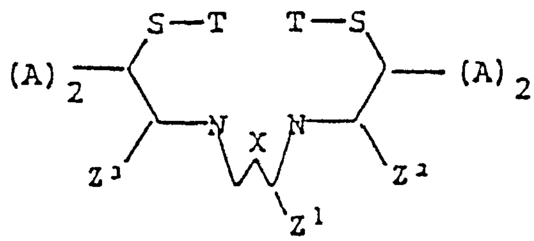

dans laquelle
l'un de $Z^1$, $Z^2$, $Z^3$, $Z^4$ est $RCW(HNV)_{n'}Y$ et les autres sont $H_2$ ou =O;
R est un radical divalent de 1 à 6 atomes de carbone et de 0 à 2 hétéroatomes;
W est =NH ou =O pourvu que le W lié à l'atome de carbone lié à Y soit $H_2$ quand Y est $-NH_2$;
V est identique à ou différent de RCW et prend les définitions de RCW;
n est 0 ou 1;
T est un groupement protecteur éliminable, un atome d'hydrogène ou un ion de métal alcalin;
Y est un groupement oxy organique formant un ester actif, $-NH_2$, $-NHNH_2$ ou un polypeptide d'au moins deux acides aminés;
X est une liaison ou un groupement méthylène ou $CHZ^4$; et les radicaux A sont identiques ou différents et sont des atomes d'hydrogène ou des groupements alkyle inférieur de 1 à 6 atomes de carbone.

**5.** Composé selon la revendication 4, dans lequel X est une liaison.

**6.** Composé selon les revendications 4 ou 5, dans lequel Y est un groupement phénoxy activé.

**7.** Procédé de préparation d'un polypeptide à marqueur radioactif, qui consiste
à combiner dans un milieu aqueux un polypeptide et un composé de formule selon la revendication 1, dans laquelle Y est le groupement labile d'un ester actif pouvant former une liaison amide ou amidine avec ledit polypeptide, ou $-NH_2$ ou $-NHNH_2$, pourvu que quand Y est $-NHNH_2$ ou $-NH_2$, ledit polypeptide possède un groupement oxo par suite de la coupure glycolique d'un sucre lié audit polypeptide et ladite combinaison se fasse dans des conditions d'amination réductrice;
ledit composé formant une liaison covalente stable avec ledit polypeptide en l'absence substantielle de radionucléide non chélaté.

**8.** Procédé de préparation d'un polypeptide à marqueur radioactif, qui consiste :
à combiner dans un milieu aqueux un composé selon l'une quelconque des revendications 4 à 6, dans lequel T est un ion de métal alcalin ou un groupement protecteur du soufre et Y est ledit polypeptide, et un radionucléide métallique sous une forme échangeable chélatée réduite;
ledit radionucléide étant alors chélaté par ledit composé; et
à laver ledit composé contenant le radionucléide pour enlever le radionucléide non chélaté lié audit composé.

**9.** Composé de formule

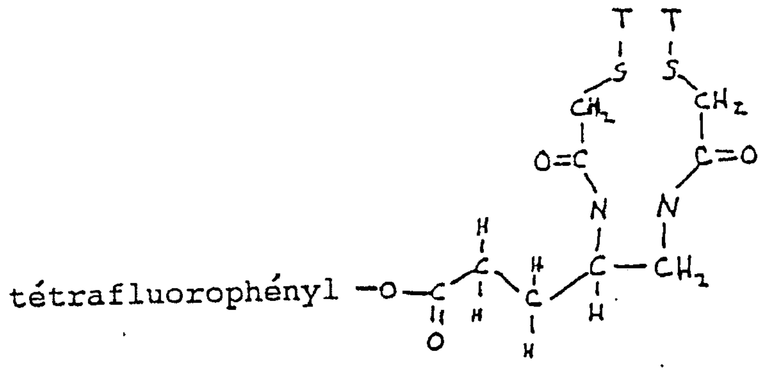

dans laquelle T est un groupement protecteur du soufre.

**10.** Composé de formule

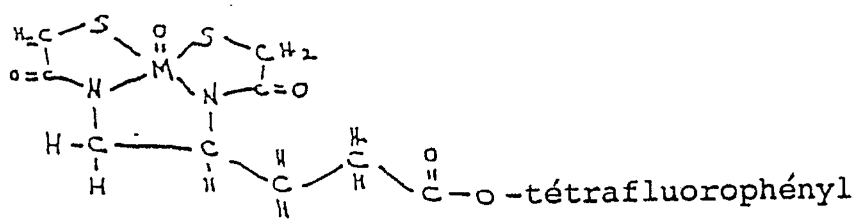

dans laquelle M représente un radionucléide de métal choisi entre Tc-99m et Re-186.

**REVENDICATIONS pour l'Etat contractant suivant: AT**

**1.** Procédé de préparation d'un composé de formule

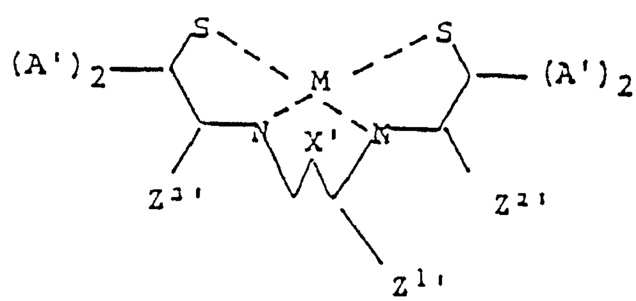

M est un ion de radionucléide de métal ou d'oxyde métallique pouvant être chélaté;
l'un de $Z^{1'}$, $Z^{2'}$, $Z^{3'}$, $Z^{4'}$ est $R'CW'(HNV)_{n'}Y'$ et les autres sont $H_2$ ou $=O$;
R' est un radical divalent aliphatique de 1 à 6 atomes de carbone et de 0 à 2 hétéroatomes;
Y' est le groupement labile d'un ester activé, $-NH_2$, $-NHNH_2$ ou un polypeptide de poids moléculaire d'au moins environ 1000;
V' a la formule R'CW';
W' est $=NH$ ou $=O$, pourvu que le groupement W' lié à l'atome de carbone lié à Y' soit $H_2$ quand Y' est $-NH_2$;
X' est une liaison, un groupement méthylène ou $CHZ^{4'}$;
les radicaux A' sont identiques ou différents et sont des atomes d'hydrogène ou des groupements alkyle inférieur ayant de 1 à 6 atomes de carbone; et
n' est 0 ou 1
comprenant l'étape consistant à chélater un ion de radionucléide de métal ou d'oxyde métallique avec un précurseur dudit composé.

**2.** Procédé selon la revendication 1, dans lequel Y' est un polypeptide.

**3.** Procédé selon la revendication 2, dans lequel Y' est une immunoglobuline ou un fragment à liaison spécifique de celle-ci.

**4.** Procédé de préparation d'un polypeptide à marqueur radioactif, qui consiste
à combiner dans un milieu aqueux un polypeptide et un composé de formule selon la revendication 1, dans laquelle Y est le groupement labile d'un ester actif pouvant former une liaison amide ou amidine avec ledit polypeptide, ou $-NH_2$ ou $-NHNH_2$, pourvu que quand Y est $-NHNH_2$ ou $-NH_2$, ledit polypeptide possède un groupement oxo par suite de la coupure glycolique d'un sucre lié audit polypeptide et ladite combinaison se fasse dans des conditions d'amination réductrice;
ledit composé formant une liaison covalente stable avec ledit polypeptide en l'absence substantielle de radionucléide non chélaté.

**5.** Procédé de préparation d'un polypeptide à marqueur radioactif, qui consiste :
à laver un composé contenant un radionucléide obtenu selon la revendication 1 pour enlever le radionucléide non chélaté lié audit composé.

**6.** Procédé selon l'une quelconque des revendications précédentes, caractérisé en ce qu'on prépare un composé de formule

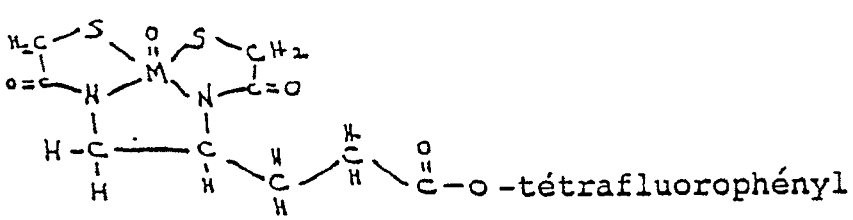

dans laquelle M représente un radionucléide de métal choisi entre Tc-99m et Re-186.

**Patentansprüche**
**Patentansprüche für folgende Vertragsstaaten: BE, CH, DE, FR, GB,IT, LI, LU, NL, SE.**

**1.** Verbindung mit der Formel:

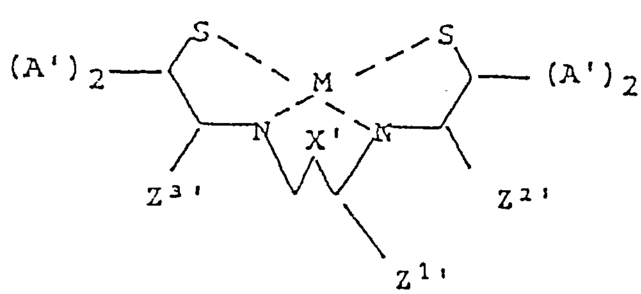

in der

M ein chelatisierbares Metall- oder Metalloxid-Radionuklidion ist;

eine der Gruppen $Z^{1'}$, $Z^{2'}$, $Z^{3'}$ und $Z^4$, $R'CW'(HNV)_n,Y'$ ist und die anderen $H_2$ oder $=O$ sind;

R' ein aliphatischer, zweibindiger Rest mit 1 bis 6 Kohlenstoffatomen und 0 bis 2 Heteroatomen ist;

Y' die austretende Gruppe eines aktiven Esters, $-NH_2$, $-NHNH_2$ oder ein Polypeptid mit einem

Molekulargewicht von mindestens ungefähr 1000 ist;

V' die Formel R'CW' aufweist;

W' =NH oder =O ist, mit der Bedingung, daß das an das mit Y' verknüpfte Kohlenstoffatom gebundene W' $H_2$ ist, wenn Y' $-NH_2$ ist;

X' eine Bindung, Methylen oder $CHZ^{4'}$ ist;

die Gruppen A', die gleich oder verschieden sein können, Wasserstoff oder niederes Alkyl mit 1 bis 6 Kohlenstoffatomen sind; und

n' 0 oder 1 ist.

2. Verbindung nach Anspruch 1, in der Y' ein Polypeptid ist.

3. Verbindung nach Anspruch 2, in der Y' ein Immunoglobin oder spezifisches bindendes Fragment derselben ist.

4. Verbindung mit der Formel:

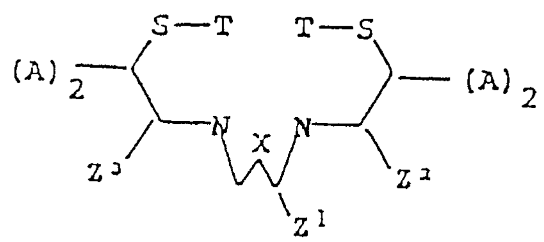

in der

eine der Gruppen $Z^1$, $Z^2$, $Z^3$ und $Z^4$ $RCW(HNV)_nY$ ist und die anderen $H_2$ oder =O sind;

R ein zweibindiger Rest mit 1 bis 6 Kohlenstoffatomen und 0 bis 2 Heteroatomen ist;

W =NH oder =O ist, mit der Bedingung, daß das an das mit Y verknüpfte Kohlenstoffatom gebundene W $H_2$ ist, das an das an Y gebundene Kohlenstoffatom gebunden ist, wenn Y $-NH_2$ ist;

V gleich oder verschieden von RCW ist und unter die Definitionen von RCW fällt;

n 0 oder 1 ist;

T eine entfernbare Schutzgruppe, Wasserstoff oder ein Alkalimetallion ist;

Y eine organische, einen aktiven Ester bildende Oxyverbindung, $-NH_2$, $-NHNH_2$ oder ein Polypeptid aus mindestens zwei Aminosäuren ist;

X eine Bindung, Methylen oder $CHZ^4$ ist; und

die Gruppen A, die gleich oder verschieden sein können, Wasserstoff oder niederes Alkyl mit 1 bis 6 Kohlenstoffatomen sind.

5. Verbindung nach Anspruch 4, in der X eine Bindung ist.

6. Verbindung nach Anspruch 4 oder 5, in der Y eine aktivierte Phenoxygruppe ist.

**7.** Verfahren zur Herstellung eines radioaktiv markierten Polypeptids mit den Merkmalen:

Zusammengeben eines Polypeptids und einer Verbindung mit der Formel gemäß Anspruch 1 in einem wäßrigen Medium, wobei Y die austretende Gruppe eines aktiven Esters, der eine Amid- oder Amidin-Verknüpfung mit dem Polypeptid bilden kann, oder $NH_2$ oder $NHNH_2$ ist, mit der Bedingung, daß, wenn Y $NHNH_2$ oder $-NH_2$ ist, das Polypeptid eine Oxo-Gruppe als Ergebnis eines Glykolspaltung eines an das Polypeptid gebundenen Zuckers aufweist und das Zusammengeben unter reduktiven Aminierungsbedingungen erfolgt;

wobei die Verbindung eine stabile, kovalente Bindung mit dem Polypeptid unter weitgehender Anwesenheit von unchelatisiertem Radionuklid bildet.

**8.** Verfahren zur Herstellung eines radioaktiv markierten Polypeptids mit den Merkmalen:

Zusammengeben einer Verbindung gemäß einem der Ansprüche 4 bis 6, in der T ein Alkalimetallion oder eine Schwefel-Schutzgruppe und Y das Polypeptid ist, und eines Metall-Radionuklids in einer reduzierten, chelatisierten, veränderlichen Form in einem wäßrigen Medium;

wobei das Radionuklid durch die Verbindung chelatisiert wird; und

Waschen der das Radionuklid enthaltenden Verbindung, um unchelatisiertes, an die Verbindung gebundenes Radionuklid zu entfernen.

**9.** Verbindung mit der Formel

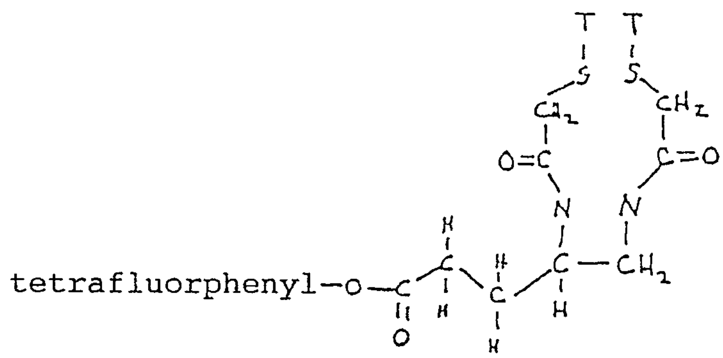

in der T eine Schwefel-Schutzgruppe ist.

**10.** Verbindung mit der Formel:

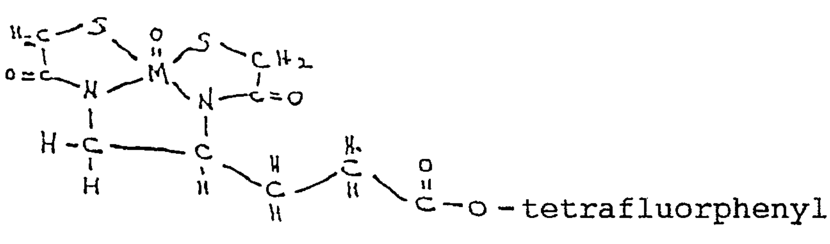

in der M ein Metall-Radionuklid, ausgewählt aus $^{99m}Tc$ und $^{186}Re$, darstellt.

**Patentansprüche für folgenden Vertragsstaat: AT**

**1.** Verfahren zur Herstellung einer Verbindung mit der Formel:

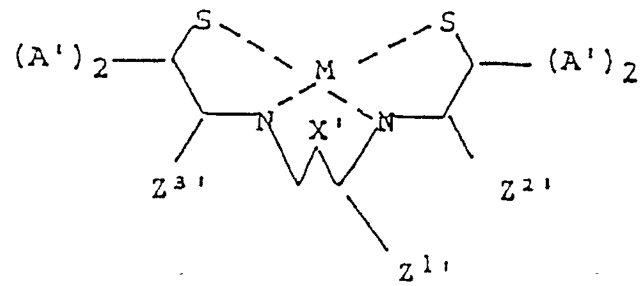

in der

M ein chelatisierbares Metall- oder Metalloxid-Radionuklidion ist;

eine der Gruppen $Z^{1'}$, $Z^{2'}$, $Z^{3'}$ und $Z^{4'}$ $R'CW'(HNV)_{n'}Y'$ ist und die anderen $H_2$ oder =O sind;

R' ein aliphatischer, zweibindiger Rest mit 1 bis 6 Kohlenstoffatomen und 0 bis 2 Heteroatomen ist;

Y' die austretende Gruppe eines aktiven Esters, $-NH_2$, $-NHNH_2$ oder ein Polypeptid mit einem Molekulargewicht von mindestens ungefähr 1000 ist;

V' die Formel R'CW' aufweist;

W' =NH oder =O ist, mit der Bedingung, daß das an das mit Y' verknüpfte Kohlenstoffatom gebundene W' $H_2$ ist, das an das an Y' gebundene Kohlenstoffatom gebunden ist, wenn Y' $-NH_2$ ist;

X' eine Bindung, Methylen oder $CHZ^{4'}$ ist;

die Gruppen A, die gleich oder verschieden sein können, Wasserstoff oder niederes Alkyl mit 1 bis 6 Kohlenstoffatomen sind; und

n' 0 oder 1 ist,

mit dem Schritt des Chelatisierens eines Metall- oder Metalloxid-Radionuklid-Ions mit einem Vorläufer der Verbindung.

**2.** Verfahren nach Anspruch 1, in dem Y' ein Polypeptid ist.

**3.** Verfahren nach Anspruch 2, dadurch gekennnzeichnet, daß Y' ein Immunoglobin oder spezifisches bindendes Fragment desselben ist.

**4.** Verfahren zur Herstellung eines radioaktiv markierten Polypeptids mit den Merkmalen:

Zusammengeben eines Polypeptids und einer Verbindung mit der Formel gemäß Anspruch 1 in einem wäßrigen Medium, wobei Y die austretende Gruppe eines aktiven Esters, der eine Amid- oder Amidin-Verknüpfung mit dem Polypeptid bilden kann, oder $NH_2$ oder $NHNH_2$ ist, mit der Bedingung, daß, wenn Y $NHNH_2$ oder $-NH_2$ ist, das Polypeptid eine Oxo-Gruppe als Ergebnis einer Glykolspaltung eines an das Polypeptid gebundenen Zuckers aufweist und das Zusammengeben unter reduktiven Aminierungsbedingungen erfolgt;

wobei die Verbindung eine stabile, kovalente Bindung mit dem Polypeptid unter weitgehender Abwesenheit von unchelatisiertem Radionuklid bildet.

**5.** Verfahren zur Herstellung eines radioaktiv markierten Polypeptids mit dem Merkmal:

Waschen einer nach Anspruch 1 erhaltenen, ein Radionuklid enthaltenden Verbindung, um unchelatisiertes, an die Verbindung gebundenes Radionuklid zu entfernen.

6. Verfahren nach einem jeden der vorherigen Ansprüche, dadurch gekennzeichnet, daß eine Verbindung mit der Formel:

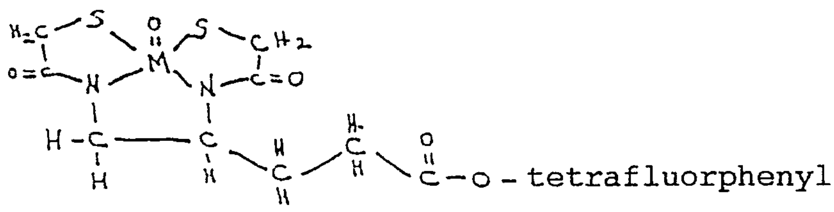

hergestellt wird,

in der M ein Metall-Radionuklid, ausgewählt aus $^{99m}Tc$ und $^{186}Re$, darstellt.